# EUROPÄISCHE PATENTANMELDUNG

(11) **EP 1 529 838 A1**
(43) Veröffentlichungstag der Anmeldung: **11.05.2005**
(21) Anmeldenummer: 03025506.1
(22) Anmeldetag: 06.11.2003
(51) Int. Cl.: C12N 5/06, G01N 33/53

(54) **Neurale Vorläufer- und Stammzellen**

(71) Anmelder: Memorec Biotec GmbH, 50829 Köln (DE)
(72) Erfinder: Bosio, Andreas, Dr., 50931 Köln (DE); Cremer, Harold, 13400 Marseille (FR); Pennartz, Sandra, 50670 Köln (DE)
(74) Vertreter: Schreiber, Christoph, Dr.

(57) **Zusammenfassung**

Zellpopulation, dadurch gekennzeichnet, dass mindestens 5% der Zellen neurale Vorläuferzellen sind, die wenigstens einen der in **Liste A** oder **Liste B** aufgeführten Marker aufweisen.

## Beschreibung

Die vorliegende Erfindung betrifft Zellpopulationen von neuralen Vorläuferzellen bzw. neuralen Stammzellen sowie Verfahren zur Isolierung entsprechender Zellen.

Der Ausgangspunkt für die Generierung der über tausend verschiedenen neuronalen und glialen Zelltypen des Nervensystems von Vertebraten sind multipotente, neurale Stammzellen des embryonalen Neuroepitheliums (Williams, B. P., Read, J. & Price, J. (1991): The generation of neurons and oligodendrocytes from a common precursor cell. *Neuron* **7**(4), 685-93), (Davis, A. A. & Temple, S. (1994): A self-renewing multipotential stem cell in embryonic rat cerebral cortex. *Nature* **372**(6503), 263-6), (Weiss, S., Dunne, C., Hewson, J., Wohl, C., Wheatley, M., Peterson, A. C. & Reynolds, B. A. (1996): Multipotent CNS stem cells are present in the adult mammalian spinal cord and ventricular neuroaxis. *J Neurosci* **16**(23), 7599-609).

In den vergangenen Jahren wurde durch verschiedene Arbeitsgruppen gezeigt, dass solche sich selbst erneuernden, multipotenten Vorläuferzellen nicht nur während der Entwicklung, sondern auch im adulten Gehirn zu finden sind (Gage, F. H. (2000): Mammalian neural stem cells. *Science* **287**(5457), 1433-8). Vor allem um die lateralen Ventrikel des Vorderhirns findet die Bildung von neuralen Vorläuferzellen lebenslang statt. Diese wandern hauptsächlich, wenn auch nicht exklusiv, in den Bulbus olfaktorius, um dort in GABA-erge Interneurone zu differenzieren.

Über die genaue Lokalisation der multipotenten Stammzellen, die dieser sekundären Neurogenese zugrunde liegen, wird derzeit noch spekuliert: Johansson et al. beschrieben ependymale Zellen entlang des Lumen der adulten, ventrikulären Zone mit den Eigenschaften multipotenter Stammzellen (Johansson, C. B., Svensson, M., Wallstedt, L., Janson, A. M. & Frisen, J. (1999b): Neural stem cells in the adult human brain. *Exp Cell Res* **253**(2), 733-6), während Doetsch et al. Astrocyten der subventrikulären Zone als multipotente Stammzellen identifizierten (Doetsch, F., Caille, I., Lim, D. A., Garcia-Verdugo, J. M. & Alvarez-Buylla, A. (1999): Subventricular zone astrocytes are neural stem cells in the adult mammalian brain. *Cell* **97**(6), 703-16). Eine absolut eindeutige Identifizierung dieser adulten Stammzellen *in vivo* ist jedoch bis heute, hauptsächlich mangels geeigneter Marker, nicht gelungen.

Neben ihrer Bedeutung im olfaktorischen System ist das therapeutische Potential der adulten Stammzellen von besonderem Interesse. Aufgrund ihrer Multipotenz weisen neurale Stammzellen bemerkenswerte Formbarkeit auf und könnten daher durch Zusatz von verschiedenen Faktoren zur Erzeugung verschiedener Neuronentypen eingesetzt werden. Die anschließende Transplantation der so entwickelten spezialisierten Zellen könnte zur Behandlung von neurologischen Krankheiten Alzheimer, Parkinson, Folgen von Schädel-Hirn-Traumata und Schlaganfall beitragen. Voraussetzung dafür ist die Charakterisierung der verschiedenen, neuralen Differenzierungsstufen sowie die Identifizierung der Faktoren, die die Differenzierungsprogramme der Stammzellen steuern. Gegenüber den embryonalen Stammzellen haben die adulten den Vorteil, dass sie erstens keine abstoßende Immunreaktion auslösen würden, weil sie dem Körper des Patienten entstammen, folglich ihre Transplantation ohne Immunsuppression erfolgen könnte, und zweitens ihre Gewinnung ethisch unbedenklich ist.

Die Erforschung der Eigenschaften neuraler Stammzellen und embronaler Stammzellen des Menschen ist aus ethischen Aspekten praktisch nicht oder nur sehr eingeschränkt möglich. Daher wurden alle explorativen Arbeiten ausgehend von Mäusen und Mauszellen durchgeführt. Wie bereits beschrieben war die Isolierung von neuralen Stammzellen bisher nicht möglich, da dieser Zelltyp nicht eindeutig charakterisiert war und keine geeigneten Marker zur Identifizierung und Anreicherung zur Verfügung standen.

Aufgabe der vorliegenden Erfindung war es daher Verfahren zu entwickeln, die eine Isolation von neuralen Vorläuferzellen und neuralen Stammzellen erlauben und entsprechende Zellpopulation, enthaltend diese Zelle bereitzustellen.

Erfindungsgemäß wird die Aufgabe gelöst durch die Identifizierung von Markern, die entsprechende Zellen aufweisen.

Marker ist ein Gen, das mit Hilfe der Serial Analysis of Genexpression (SAGE) in entsprechenden Zellen gefunden wird.

Methodisch beruht SAGE auf der Isolierung von 14 bp großen DNA Fragmenten (*Tags*), die jeweils charakteristisch für eine mRNA-Spezies sind. Die *Tags,* repräsentativ für alle in der zu untersuchenden Zelle vorliegenden mRNA Moleküle, werden zu langen Polymeren verbunden, die im letzten Schritt der Methode sequenziert werden. Die Frequenz, mit der ein Tag sequenziert wird, ist direkt proportional zur Kopienzahl der mRNA-Moleküle im untersuchten Ausgangsmaterial (Velculescu, V. E., Zhang, L., Vogelstein, B. & Kinzler, K. W. (1995): Serial analysis of gene expression. *Science* **270**(5235), 484-7). Durch die computerunterstützte Auswertung der Sequenzdaten entsteht ein digitales Expressionsprofil, das beliebig oft und ohne zusätzliche Laborarbeit mit Expressionsprofilen anderer Gewebe verglichen werden kann (Meta-Analyse).

Den so identifizierten Gene sind eindeutigen Nummern zugeordnet, die beispielsweise als SAGEmap von National Center for Biotechnology Information (NCBI) bereitgestellt werden (www.ncbi.nlm.nin.gov/SAGE).

Gegenstand der Erfindung sind zum einen Zellpopulationen, bei denen mindestens 5% der Zellen neurale Vorläuferzellen sind, die wenigstens einen der in **Liste A** oder **Liste B** aufgeführten Marker aufweisen.

Bevorzugt weisen entsprechende neurale Vorläuferzellen wenigstens zwei, drei, vier oder fünf der in **Liste A** oder **B** aufgeführten Marker auf.

In bevorzugten Ausführungsformen weisen entsprechende neurale Vorläuferzellen keinen der in **Liste C** aufgeführten Marker auf.

Bevorzugt ist der Gehalt an neuralen Vorläuferzellen in der Zellpopulation hoch, d.h. mindestens 10%, bevorzugt mindestens 25%, noch mehr bevorzugt mehr als 50% und am meisten bevorzugt über 90%.

Entsprechende neurale Vorläuferzellen sind vorzugsweise aus Hirngewebe erhältlich.

In einer Ausführungsform handelt es sich dabei um eine murine Zellpopulation.

Gegenstand der Erfindung ist auch ein Verfahren zur Isolierung einer entsprechenden Zellpopulation mit folgenden Schritten:
entweder
- Entnahme einer Probe aus dem Hirn
- Isolieren der neuralen Vorläuferzellen unter Verwendung der angegebenen Marker
oder
- Differenzierung von embryonalen Stammzellen zu neuralen Vorläuferzellen,
- Isolieren der neuralen Vorläuferzellen unter Verwendung der angegebenen Marker
oder
- Trans-Differenzierung von adulten, nicht neuralen Stammzellen zu neuralen Vorläuferzellen,
- Isolieren der neuralen Vorläuferzellen unter Verwendung der angegebenen Marker
oder
- Differenzierung von adulten, neuralen Stammzellen zu neuralen Vorläuferzellen,
- Isolieren der neuralen Vorläuferzellen unter Verwendung der angegebenen Marker
oder
- Differenzierung von immortalisierten Zellen zu neuralen Vorläuferzellen,
- Isolieren der neuralen Vorläuferzellen unter Verwendung der angegebenen Marker.

"Unter Verwendung der angegebenen Marker" bedeutet, dass die Zellen isoliert werden, die positiv für mindestens einen der Marker aus der **Liste A** und **B** sind, wobei mehrere positive Marker und die Abwesenheit von Markern der **Liste C** bevorzugt werden. Die Isolierung kann beispielsweise durch FACS Analyse erfolgen. Die durch die Verfahren erhältlichen Zellen sind ebenfalls Gegenstand der Erfindung.

Ein weiterer Gegenstand der Erfindung ist die Verwendung mindestens eines Markers ausgewählt aus der **Liste A** oder **Liste B** zu Identifizierung oder Isolierung von neuralen Vorläuferzellen.

Gegenstand ist weiterhin ein Antikörper gegen einen Marker aus der **Liste A, B** oder **C**, ein Diagnostikmittel enthaltend mindestens einen, bevorzugt zwei oder mehr Substanzen zur Erkennung der Marker der **Liste A, B** oder **C** sowie ein Arzneimittel enthaltend die erfindungsgemäße Zellpopulation.

Solche Arzneimittel könnten wie oben dargestellt zur Behandlung von neurologischen Krankheiten wie Alzheimer, Parkinson, Folgen von Schädelhirntraumata oder Schlaganfall eingesetzt werden.

Ein weiterer Gegenstand ist eine Zellpopulation, bei der mindestens 5% der Zellen neurale Stammzellen sind, die wenigstens einen der in **Liste D** oder **Liste E** aufgeführten Marker aufweisen.

Vorzugsweise weisen entsprechende neurale Stammzellen mindestens zwei, bevorzugt mindestens drei, mindestens vier und noch mehr bevorzugt mindestens fünf der in **Liste D** oder **Liste E** aufgeführten Marker auf.

In besonders bevorzugten Ausführungsformen weisen entsprechende neurale Stammzellen keinen der in **Liste A** oder **Liste C** aufgeführten Marker auf.

Der Gehalt an neuralen Stammzellen in der Zellpopulation ist möglichst hoch, bevorzugt mindestes 10%, mehr bevorzugt mindestes 25%, mindestens 50%, und am meisten bevorzugt mindestens 90%.

Entsprechende Zellpopulation sind aus Hirngewebe erhältlich. In einer Ausführungsform handelt es sich um eine murine Zellpopulation.

Gegenstand ist weiterhin ein Verfahren zur Isolierung der Zellpopulation. Dies ist erhältlich entweder durch
- Entnahme einer Probe aus dem Hirn
- Isolieren der neuralen Stammzellen unter Verwendung der angegebenen Marker
oder
- Differenzierung von embryonalen Stammzellen zu neuralen Stammzellen,
- Isolieren der neuralen Stammzellen unter Verwendung der angegebenen Marker
oder
- Trans-Differenzierung von adulten, nicht neuralen Stammzellen zu neuralen Stammzellen,
- Isolieren der neuralen Stammzellen unter Verwendung der angegebenen Marker
oder
- De-Differenzierung von adulten, neuralen Vorläuferzellen zu neuralen Stammzellen,
- Isolieren der neuralen Stammzellen unter Verwendung der angegebenen Marker
oder
- Differenzierung von immortalisierten Zellen zu neuralen Stammzellen,
- Isolieren der neuralen Stammzellen unter Verwendung der angegebenen Marker.

Die Isolierung erfolgt wie oben bei den neuralen Vorläuferzellen angegeben. Auch die auf diesem Wege erhältlichen neuralen Stammzellen sind Gegenstand der Erfindung.

Gegenstand der Erfindung ist weiterhin ein Antikörper gegen einen Marker aus der **Liste D, E**, ein Diagnostikmittel enthaltend mindestens einen, bevorzugt zwei oder mehr Substanzen zur Erkennung der Marker der **Liste D, E, A** oder **C** sowie ein Arzneimittel enthaltend die erfindungsgemäße Zellpopulation.

Solche Arzneimitteln können wie dargestellt zur Behandlung von neuronalen Krankheiten wie Alzheimer, Parkinson, Folgen von Schädelhirntraumata oder Schlaganfall eingesetzt werden.

### Beispiele

### A. Isolierung von embryonaler Stammzellen

Murine embryonale Stammzellen proliferieren klonal *in vitro* und sind aus diesem Grunde in großer Menge und hochreiner Form isolierbar. Nach dem Stand der Technik werden diese in Anwesenheit von LIF auf primären embryonalen Fibroblasten gehalten und regelmäßig durch die Generierung von hochgradig keimbahnkompetenten chimären Mäusen auf ihre Qualität überprüft. Unter normalen Kulturbedingungen beträgt das Verhältnis ES-Zellen zu kontaminierenden Fibroblasten etwa 200:1. Um auch diese minoritäre Komponente zu eliminieren, wurden die ES-Zellen vor der RNA-Päparation für zwei Passagen (vier Tage) auf gelatinisierten Kulturplatten bei erhöhter LIF-Konzentration gehalten. Dies ermöglicht eine Reduktion der kontaminierenden Fibroblasten auf etwa 0,01% der Gesamtpopulation.

### B. Isolierung von neuronalen Vorläuferzellen aus dem adulten Mausgehirn.

In der subventrikulären Zone des adulten Vorderhirns von Vertebraten werden permanent große Mengen von neuralen Vorläuferzellen gebildet (wahrscheinlich < 50000 Zellen/ Tag). Diese Zellen benutzen einen präzise definierten Migrationsweg und eine spezielle Form der Translokation (*Chain migration*) um in den Bulbus olfaktorius zu gelangen. Im Bulbus olfaktorius angelangt differenzieren diese Vorläuferzellen normalerweise in inhibitorische (GABA-erge) Interneurone. Unter bestimmten experimentellen Bedingungen wurde ihre Differenzierung in Oligodendrozyten und Astrozyten gezeigt.

Neurale Vorläufer, die einen Differenzierungszustand zwischen einer neuralen Stammzelle und einem terminal differenzierten Neuron repräsentieren, exprimieren spezifisch eine Form des neuralen Zelladhäsionsmoleküls NCAM, die eine spezielle post-translationelle Modifikation aufweist. Diese Modifikation besteht aus der Glykosylierung des Proteins mit a-2,8 verknüpfter Polysialylsäure (PSA). Ein spezifischer Antikörper gegen dieses Glykoepitop (Chazal *et al.,* 2000) erlaubte die hochreine Isolierung der Zielpopulation aus dissoziierten Vorderhirngewebe durch FACS (*Fluorescence Activated Cell Sorting*).

### C. Molekulargenetische Analyse

Embryonale Stammzellen und neuronale Vorläuferzellen wurden in einem genomweiten Screen mit der Methode SAGE (*Serial Analysis of Gene Expression*) analysiert.

Die Genexpressionsprofile der beiden Zell-Populationen wurden unter Anwendung bioinformatischer Verfahrensweisen mit Maus-Hirn-SAGE-Datenbanken verglichen, um molekulare Marker zu identifizieren, die charakteristisch für embryonale Stammzellen und neuronale Vorläuferzellen sind.

Mit Hilfe der Microarray technologie wurde die Expression der Gene bestätigt.

Durch *in situ*-Hybridisierung in Maushirn und an embryonalen Stammzellen wurde die zelluläre Lokalisation einiger der identifizierten Gene bestimmt. Diese Ergebnisse belegen, dass spezifische Markergene identifiziert werden konnten.

### Liste A: Positivmarker neurale Vorläuferzellen (1.) und Negativmarker 2 neurale Stammzellen;

### ES-Zellen -; PSA-NCAM +; Adult brain -

- Mm.8884: nuclear factor of kappa light chain gene enhancer in B-cells inhibitor, alpha
- Mm.8180: lymphocyte antigen 6 complex, locus A
- Mm.6238: SRY-box containing gene 11
- Mm.517: (Manual) Manic fringe protein, putative secreted glycosyltransferase, notch modulator
- Mm.4919: DNA segment, human D4S114
- Mm.4727: seizure related gene 6
- Mm.45769: ESTs
- Mm.44490: RIKEN cDNA 6330415M09 gene
- Mm.42948: peroxiredoxin 2
- Mm.4022: RIKEN cDNA 1110033C18 gene
- Mm.3940: lethal giant larvae homolog
- Mm.37835: ribosomal protein L7
- Mm.3779: RIKEN cDNA 2300006C11 gene
- Mm.340: high mobility group box 3
- Mm.32902: ESTs, Weakly similar to S26689 hypothetical protein hc1 - mouse
- Mm.3268: ubiquitin-conjugating enzyme E2I
- Mm.31436: myeloid ecotropic viral integration site-related gene 1
- Mm.297: actin, beta, cytoplasmic
- Mm.29558: expressed sequence AI426163
- Mm.29014: T-cell lymphoma invasion and metastasis 2
- Mm.28842: chloride channel 3
- Mm.28824: Mus musculus, clone IMAGE:4504748, mRNA
- Mm.28275: RNA binding motif protein, X chromosome
- Mm.28149: RIKEN cDNA 3110003A17 gene
- Mm.28148: chromobox homolog 3 (Drosophila HP1 gamma)
- Mm.27816: hexosaminidase B
- Mm.2769: MARCKS-like protein
- Mm.22171: calponin 3, acidic
- Mm.220923: RIKEN cDNA 6530406007 gene
- Mm.21740: heterogeneous nuclear ribonucleoprotein H1
- Mm.206085: expressed sequence AI854782
- Mm.205996: EST AA087124
- Mm.200858: RIKEN cDNA 2410129E14 gene
- Mm.199500: expressed sequence AI844617
- Mm.195901: ribosomal protein L35a
- Mm.194965: EST
- Mm.19101: DEAD (aspartate-glutamate-alanine-aspartate) box polypeptide 5
- Mm.19016: drebrin 1
- Mm.18789: SRY-box containing gene 4
- Mm.186740: ESTs
- Mm.18516: H3 histone, family 3B
- Mm.181959: early growth response 1
- Mm.181847: prefoldin 5
- Mm.16421: high mobility group box 1
- Mm.15534: interleukin 1 alpha
- Mm.13725: Paneth cell enhanced expression
- Mm.12871: doublecortin
- Mm.127662: ESTs
- Mm.12412: Mus musculus, Similar to RIKEN cDNA 2810407E23 gene, clone IMAGE:4489006, mRNA, partial cds

### Liste B: Positivmarker neurale Vorläuferzellen (2.);

### ES-Zellen -/+; PSA-NCAM +; Adult brain -

- Mm.911: high mobility group nucleosomal binding domain 2
- Mm.89136: H3 histone, family 3A
- Mm.741: fatty acid binding protein 5, epidermal
- Mm.7286: C-terminal binding protein 1
- Mm.7141: proliferating cell nuclear antigen
- Mm.6840: RIKEN cDNA 5730507C05 gene
- Mm.6787: splicing factor, arginine/serine-rich 3 (SRp20)
- Mm.6417: CD24a antigen
- Mm.6343: nucleophosmin 1
- Mm.482: Jun oncogene
- Mm.43871: expressed sequence AW046487
- Mm.43213: RIKEN cDNA 9030402K04 gene
- Mm.42767: ribosomal protein S17
- Mm.4269: transcription factor 4
- Mm.40715: RIKEN cDNA 1110038H03 gene
- Mm.40715: RIKEN cDNA 1110038H03 gene
- Mm.4071: laminin receptor 1 (67kD, ribosomal protein SA)
- Mm.4025: nuclear factor I/B
- Mm.372: ribosomal protein S26
- Mm.3487: ribosomal protein L30
- Mm.3381: ribosomal protein S8
- Mm.31051: RIKEN cDNA 2610003J05 gene
- Mm.30120: ribosomal protein S27-like
- Mm.30011: ribosomal protein S23
- Mm.29911: RIKEN cDNA 3200001M24 gene
- Mm.2966: isocitrate dehydrogenase 2 (NADP+), mitochondrial
- Mm.29580: superiorcervical ganglia, neural specific 10
- Mm.2958: expressed sequence AI843786
- Mm.28985: ribosomal protein L27
- Mm.28869: ESTs
- Mm.27927: heterogeneous nuclear ribonucleoprotein A1
- Mm.27669: small nuclear ribonucleoprotein E
- Mm.2756.: high mobility group nucleosomal binding domain 1
- Mm.27141: Rac GTPase-activating protein 1
- Mm.2591: RNA binding motif protein 3
- Mm.24083: Mus musculus, Similar to TAR DNA binding protein, clone MGC: 19284 IMAGE:4016437, mRNA, complete cds
- Mm.219668: RIKEN cDNA 2610209F03 gene
- Mm.21841: splicing factor, arginine/serine-rich 2 (SC-35)
- Mm.218240: Mus musculus, clone IMAGE:5342828, mRNA, partial cds
- Mm.21740: heterogeneous nuclear ribonucleoprotein H1
- Mm.213020: (Manual) 60S ribosomal protein L32 (RPL32)
- Mm.2115: heterogeneous nuclear ribonucleoprotein U
- Mm.196611: synapsin I
- Mm.19187: prothymosin alpha
- Mm.18789: SRY-box containing gene 4
- Mm.186499: ESTs, Weakly similar to immunoglobulin superfamily containing leucinerich repeat
- Mm.18516: H3 histone, family 3B
- Mm.180873: RIKEN cDNA 2510019J09 gene
- Mm.1775: hematological and neurological expressed sequence 1
- Mm.1703: tubulin, beta 5
- Mm.16775: ribosomal protein S24
- Mm.16767: heterogeneous nuclear ribonucleoprotein A2/B1
- Mm.16596: B-cell translocation gene 1, anti-proliferative
- Mm.148973: RIKEN cDNA 3010025E17 gene
- Mm.142872: heterogeneous nuclear ribonucleoprotein K
- Mm.142729: thymosin, beta 4, X chromosome
- Mm.140380: ribosomal protein L23
- Mm.140: protein phosphatase 1, regulatory (inhibitor) subunit 14B
- Mm. 12858: eukaryotic translation initiation factor 4A1

### Liste C: Negativmarker 1 neurale Stammzellen und Negativmarker neurale Vorläuferzellen;

### ES-Zellen -; PSA-NCAM -; Adult brain +

- Mm.98: proteasome (prosome, macropain) subunit, beta type 6
- Mm.9745: lactate dehydrogenase 2, B chain
- Mm.970: creatine kinase, mitochondrial 1, ubiquitous
- Mm.891: kinesin family member C2
- Mm.88833: Mus musculus strain ILS K-Cl cotransporter (Slc12a5) mRNA, complete cds
- Mm.87027: BM88 antigen
- Mm.8688: RIKEN cDNA 0610011B04 gene
- Mm.86654: microtubule-associated protein 6
- Mm.848: testis expressed gene 261
- Mm.806: CD 81 antigen
- Mm.80123: ESTs, Weakly similar to simple repeat sequence-containing transcript
- Mm.7729: aldolase 3, C isoform
- Mm.7420: tubulin, beta 4
- Mm.7363: beta-spectrin 3
- Mm.726: basigin
- Mm.7089: necdin
- Mm.667: glutathione S-transferase, mu 5
- Mm.6660: small inducible cytokine A27
- Mm.6586: Mus musculus, clone MGC:6299 IMAGE:2654341, mRNA, complete cds
- Mm.6565: FK506 binding protein 8 (38 kDa)
- Mm.65337: Mus musculus, clone MGC:28924 IMAGE:3481738, mRNA, complete cds
- Mm.648: prion protein
- Mm.638: ESTs
- Mm.544: phosphoprotein enriched in astrocytes 15
- Mm.5264: ESTs, Highly similar to FEZ1_RAT FASCICULATION AND ELONGATION PROTEIN ZETA 1 (ZYGIN I)
- Mm.5259: (Manual assignment) probably myelin-associated oligodendrocyte basic protein MOBP
- Mm.5249: copine 6
- Mm.52: RIKEN cDNA 1810033A19 gene
- Mm.5195: complexin 1
- Mm.5153: neurotensin receptor 2
- Mm.5023: Purkinje cell protein 4
- Mm.4923: ESTs
- Mm.4921: glutamate receptor, ionotropic, AMPA2 (alpha 2)
- Mm.4920: glutamate receptor, ionotropic, AMPA1 (alpha 1)
- Mm.4870: synaptosomal-associated protein, 91 kDa
- Mm.4857: calcium/calmodulin-dependent protein kinase II, beta
- Mm.4762: kinesin heavy chain member 1A
- Mm.4705: (Manual) probably in far 3'-UTR of complexin-2 cDNA
- Mm.46764: RIKEN cDNA 4833409J18 gene
- Mm.4657: amyloid beta (A4) precursor protein-binding, family A, member 2
- Mm.4651: kinesin-associated protein 3
- Mm.45951: RIKEN cDNA 1200016B17 gene
- Mm.4550: ATPase, Na+/K+ transporting, beta 1 polypeptide
- Mm.4550: ATPase, Na+/K+ transporting, beta 1 polypeptide
- Mm.4537: NADH dehydrogenase (ubiquinone) 1 beta subcomplex, 9
- Mm.44355: RIKEN cDNA 6430514L14 gene
- Mm.4435: synaptosomal-associated protein, 25 kDa
- Mm.44244: open reading frame 12
- Mm.44107: ESTs
- Mm.44101: Mus musculus, ATPase, Na+K+ transporting, alpha 3 subunit, clone MGC:27631 IMAGE:4506376, mRNA, complete cds
- Mm.4383: myc box dependent interacting protein 1
- Mm.43786: cytochrome c oxidase, subunit VIIc
- Mm.43749: RIKEN cDNA 3100001N19 gene
- Mm.43721: small nuclear ribonucleoprotein N
- Mm.43587: hippocalcin
- Mm.43415: cytochrome c oxidase, subunit VI a, polypeptide 1
- Mm.4339: laminin, alpha 5
- Mm.43330: RIKEN cDNA 0610025G13 gene
- Mm.43278: olfactomedin 1
- Mm.43278: olfactomedin 1
- Mm.4296: synovial sarcoma translocation, Chromosome 18
- Mm.42949: RIKEN cDNA 1110012005 gene
- Mm.42948: peroxiredoxin 2
- Mm.42829: selenoprotein W, muscle 1
- Mm.4266: integral membrane protein 2B
- Mm.4266: integral membrane protein 2B
- Mm.4263: cystatin C
- Mm.425: histidine triad nucleotide binding protein
- Mm.42255: ATPase, Ca++ transporting, cardiac muscle, slow twitch 2
- Mm.41926: NADH dehydrogenase (ubiquinone) 1 alpha subcomplex, 4
- Mm.41925: RIKEN cDNA 1810034B16 gene
- Mm.41918: RIKEN cDNA 1110063G11 gene
- Mm.41911: cytochrome P450, 46 (cholesterol 24-hydroxylase)
- Mm.41893: RIKEN cDNA 6330408G06 gene
- Mm.41791: glycoprotein m6b
- Mm.41752: expressed sequence AI847934
- Mm.41735: RIKEN cDNA 2300004C15 gene
- Mm.41719: RIKEN cDNA 2610507A21 gene
- Mm.41711: Mus musculus, clone IMAGE:3499845, mRNA, partial cds
- Mm.41694: ESTs
- Mm.41692: ESTs, Weakly similar to F59F4.2.p
- Mm.41642: regulator of G-protein signaling 4
- Mm.41630: RIKEN cDNA 0710001E10 gene
- Mm.41604: ESTs, Weakly similar to VAV3_MOUSE VAV-3 PROTEIN
- Mm.41603: expressed sequence AI891706
- Mm.41603: expressed sequence AI891706
- Mm.41602: RIKEN cDNA 3110050O07 gene
- Mm.41602: RIKEN cDNA 3110050O07 gene
- Mm.4137: chromogranin A
- Mm.41354: ESTs
- Mm.41277: RIKEN cDNA 1110020M21 gene
- Mm.41248: ESTs
- Mm.41190: RIKEN cDNA 1700112L09 gene
- Mm.40863: expressed sequence AW049870
- Mm.40738: RIKEN cDNA 2900072M03 gene
- Mm.40621: ESTs, Moderately similar to Y552_HUMAN HYPOTHETICAL PROTEIN KIAA0552
- Mm.40472: expressed sequence AI835002
- Mm.40443: RIKEN cDNA 4930488B01 gene
- Mm.40124: phosphodiesterase 10A
- Mm.40059: ESTs, Weakly similar to SP62 MOUSE SPLICEOSOME ASSOCIATED PROTEIN 62
- Mm.39857: RIKEN cDNA 2900074L19 gene
- Mm.39803: expressed sequence AI841080
- Mm.39752: RIKEN cDNA 2900041A09 gene
- Mm.3974: ubiquitin specific protease 4 (proto-oncogene)
- Mm.39548: expressed sequence AI839779
- Mm.3951: thymus cell antigen 1, theta
- Mm.3915: myelin-associated oligodendrocytic basic protein
- Mm.39040: myelin and lymphocyte protein, T-cell differentiation protein
- Mm.38994: RIKEN cDNA 2600001N01 gene
- Mm.38993: calsyntenin 1
- Mm.38551: calcium binding protein 1
- Mm.38469: amyloid beta (A4) precursor protein-binding, family B, member 1
- Mm.38438: RIKEN cDNA 1200009K17 gene
- Mm.38421: (Manual assignment) ATPase, Na+K+ transporting, alpha polypeptide
- Mm.38421: (Manual assignment) ATPase, Na+K+ transporting, alpha polypeptide
- Mm.3840: flotillin 2
- Mm.38248: sialyltransferase 9 (CMP-NeuAc:lactosylceramide alpha-2,3-sialyltransferase)
- Mm.38036: ESTs, Moderately similar to NX1A_MOUSE_2
- Mm.38036: ESTs, Moderately similar to NX1A_MOUSE_2
- Mm.37462: ESTs, Weakly similar to CA11 RAT COLLAGEN ALPHA 1(I) CHAIN
- Mm.37214: transferrin
- Mm.36275: DNA segment, Chr 11, Brigham & Women's Genetics 0517 expressed
- Mm.3624: guanylate kinase 1
- Mm.35837: RIKEN cDNA 2510006D16 gene
- Mm.35837: RIKEN cDNA 2510006D16 gene
- Mm.3544: calcium channel, voltage-dependent, beta 3 subunit
- Mm.35439: secreted acidic cysteine rich glycoprotein
- Mm.35270: Ly6/neurotoxin 1
- Mm.3479: ATPase, H+ transporting, lysosomal 21kDa, V0 subunit B
- Mm.34695: actin related protein 2/3 complex, subunit 1A (41 kDa)
- Mm.34246: calmodulin 1
- Mm.3363: prosaposin
- Mm.3360: tyrosine 3-monooxygenase/tryptophan 5-monooxygenase activation protein, zeta polypeptide
- Mm.33117: ESTs
- Mm.3308: tyrosine 3-monooxygenase/tryptophan 5-monooxygenase activation protein, eta polypeptide
- Mm.3292: glutamate receptor, ionotropic, NMDA1 (zeta 1)
- Mm.3229: ribosomal protein L26
- Mm.32191: gamma-aminobutyric acid (GABA-B) receptor, 1
- Mm.31395: carboxypeptidase E
- Mm.3123: cornichon-like (Drosophila)
- Mm.31025: RIKEN cDNA 2310015K15 gene
- Mm.30412: RIKEN cDNA 5430400P17 gene
- Mm.30355: (Manual) KIF5A Neuronal Kinesin heavy chain
- Mm.30266: hemoglobin, beta adult major chain
- Mm.30266: hemoglobin, beta adult major chain
- Mm.30206: ATPase, H+ transporting, lysosomal 34kD, V1 subunit D
- Mm.30156: protease, serine, 11 (Igf binding)
- Mm.30155: ATPase, H+ transporting, lysosomal 16kD, V0 subunit C
- Mm.30150: RIKEN cDNA 1010001M12 gene
- Mm.30126: membrane interacting protein of RGS16
- Mm.30085: aldo-keto reductase family 1, member A4 (aldehyde reductase)
- Mm.30072: cytochrome c oxidase subunit VIIa polypeptide 2-like
- Mm.30059: myristoylated alanine rich protein kinase C substrate
- Mm.29976: septin 5
- Mm.29965: RIKEN cDNA 2410104119 gene
- Mm.29947: serine/threonine kinase 11
- Mm.29939: RIKEN cDNA 1010001N11 gene
- Mm.29937: (Manual assignment) polymorphism of Mm.29937 ESTs, Weakly similar to predicted using Genefinder
- Mm.29921: RAS protein-specific guanine nucleotide-releasing factor 1
- Mm.2992: (Manual assignment) MBP myelin basic protein
- Mm.29870: integral membrane protein 3
- Mm.29867: NADH dehydrogenase (ubiquinone) 1 alpha subcomplex 2
- Mm.29857: (Manual) Neurogranin
- Mm.29852: Mus musculus, clone IMAGE:5102170, mRNA, partial cds
- Mm.29846: Mus musculus, Similar to NDRG family, member 4, clone MGC:7067 IMAGE: 3156802, mRNA, complete cds
- Mm.29842: NADH dehydrogenase flavoprotein 1
- Mm.29823: microsomal glutathione S-transferase 3
- Mm.29807: ubiquitin carboxy-terminal hydrolase L1
- Mm.29807: ubiquitin carboxy-terminal hydrolase L1
- Mm.29771: ATPase, H+ transporting, lysosomal 70kD, V1 subunit A, isoform 1
- Mm.29717: 3-monooxgenase/tryptophan 5-monooxgenase activation protein, gamma polypeptide
- Mm.29711: adrenergic receptor kinase, beta 1
- Mm.297: actin, beta, cytoplasmic
- Mm.29633: RIKEN cDNA 1810008021 gene
- Mm.29600: Mus musculus, clone IMAGE:3964267, mRNA
- Mm.2948: H2-K region expressed gene 2
- Mm.29477: SCAN domain-containing 1
- Mm.29415: RIKEN cDNA 1810011001 gene
- Mm.29362: expressed sequence AI414999
- Mm.29344: tumor differentially expressed 1, like
- Mm.29330: expressed sequence AI853543
- Mm.29230: RIKEN cDNA 1500017E18 gene
- Mm.29227: RIKEN cDNA 2300002D11 gene
- Mm.29205: bruno-like 4, RNA binding protein (Drosophila)
- Mm.29205: bruno-like 4, RNA binding protein (Drosophila)
- Mm.2918: megakaryocyte-associated tyrosine kinase
- Mm.29141: RIKEN cDNA 0710008N11 gene
- Mm.29124: phosphatidic acid phosphatase type 2B
- Mm.29075: (Manual) Reticulon 1 protein, major internal tag
- Mm.29027: SPARC-like 1 (mast9, hevin)
- Mm.29027: SPARC-like 1 (mast9, hevin)
- Mm.2902: protein tyrosine phosphatase, receptor-type, N
- Mm.28955: RIKEN cDNA 4930570C03 gene
- Mm.28650: RAB6, member RAS oncogene family
- Mm.28650: RAB6, member RAS oncogene family
- Mm.28643: vesicle-associated membrane protein 2
- Mm.28561: protein kinase C, zeta
- Mm.28518: type I transmembrane protein Fn14
- Mm.28357: microtubule-associated protein 1 light chain 3
- Mm.2815: RIKEN cDNA 1110021H02 gene
- Mm.28107: ectonucleotide pyrophosphatase/phosphodiesterase 2
- Mm.28058: NADH dehydrogenase (ubiquinone) 1 beta subcomplex 5
- Mm.27886: RIKEN cDNA 2410011G03 gene

- Mm.27608: Mus musculus, Similar to chromosome 9 open reading frame 16, clone MGC:19388 IMAGE:2812475, mRNA, complete cds
- Mm.2755: calbindin 2
- Mm.27499: RIKEN cDNA 2010004E11 gene
- Mm.27407: RecQ protein-like
- Mm.27256: discs, large homolog 4 (Drosophila)
- Mm.2720: mitogen activated protein kinase 8 interacting protein
- Mm.27114: RIKEN cDNA 0610043B10 gene
- Mm.27087: RIKEN cDNA 2010012C24 gene
- Mm.27005: visinin-like 1
- Mm.26633: PH domain containing protein in retina 1
- Mm.26633: PH domain containing protein in retina 1
- Mm.26550: phosphofructokinase, muscle
- Mm.2645: eukaryotic translation elongation factor 1 alpha 2
- Mm.2635: pyruvate kinase 3
- Mm.2619: cholecystokinin
- Mm.25849: RIKEN cDNA 2010003014 gene
- Mm.25738: RIKEN cDNA 2900002P20 gene
- Mm.25228: ring finger protein 11
- Mm.25203: NCK-associated protein 1
- Mm.2496: internexin neuronal intermediate filament protein, alpha
- Mm.24482: RIKEN cDNA 5730460C18 gene
- Mm.2446: synaptotagmin 4
- Mm.24376: Mus musculus mRNA for calsyntenin-3 (Cs3 gene)
- Mm.2411: Ras-GTPase-activating protein (GAP<120>) SH3-domain binding protein 2
- Mm.24092: N-ethylmaleimide sensitive fusion protein
- Mm.24092: N-ethylmaleimide sensitive fusion protein
- Mm.2400: glutathione peroxidase 4
- Mm.2397: synaptophysin
- Mm.23826: phosphotyrosyl phosphatase activator
- Mm.2381: amyloid beta (A4) precursor-like protein 1
- Mm.2338: glutamine synthetase
- Mm.2338: glutamine synthetase
- Mm.2326: macrophage migration inhibitory factor
- Mm.2319: Scgn10 like-protein
- Mm.23023: RIKEN cDNA 1500009C09 gene
- Mm.23002: RIKEN cDNA 5330410G16 gene
- Mm.22699: selenoprotein P, plasma, 1
- Mm.22637: RIKEN cDNA 0910001L24 gene
- Mm.22597: RIKEN cDNA 2310042E05 gene
- Mm.22473: Rab acceptor 1 (prenylated)
- Mm.22149: succinate dehydrogenase complex, subunit A, flavoprotein (Fp)
- Mm.2214: septin 4
- Mm.220966: reticulon 4
- Mm.220898: calmodulin 3
- Mm.220885: neurochondrin
- Mm.2206: NADH dehydrogenase (ubiquinone) flavoprotein 2
- Mm.219776: RIKEN cDNA 1110001E17 gene
- Mm.218848: RIKEN cDNA 3010002G01 gene
- Mm.218764: guanine nucleotide binding protein 13, gamma
- Mm.218611: receptor (calcitonin) activity modifying protein 2
- Mm.21743: malate dehydrogenase, mitochondrial
- Mm.216438: Mus musculus, clone IMAGE:5068657, mRNA, partial cds
- Mm.216240: Mus musculus, clone IMAGE:3594799, mRNA
- Mm.21485: RIKEN cDNA 2610102M01 gene
- Mm.214549: Mus musculus, Similar to vesicle-associated calmodulin-binding protein, clone MGC:28873 IMAGE:4527857, mRNA, complete cds
- Mm.2133: centaurin, gamma 3
- Mm.212672: S100 protein, beta polypeptide, neural
- Mm.212516: RIKEN cDNA 2900002L20 gene
- Mm.21251: deleted in polyposis 1
- Mm.21162: genes associated with retinoid-IFN-induced mortality 19
- Mm.2108: transthyretin
- Mm.21071: ADP-ribosylation-like 2
- Mm.21069: RIKEN cDNA 0610007A03 gene
- Mm.20964: guanine nucleotide binding protein, alpha o
- Mm.20964: guanine nucleotide binding protein, alpha o
- Mm.2082: apolipoprotein D
- Mm.206218: Mus musculus, Similar to hypothetical protein FLJ22237, clone MGC:27683 IMAGE:4913322, mRNA, complete cds
- Mm.2060: RIKEN cDNA 2900010105 gene
- Mm.20472: vertebrate homolog of C. elegans Lin-7 type 2
- Mm.203939: expressed sequence AI256814
- Mm.203924: expressed sequence AW259572
- Mm.203921: expressed sequence AI850305
- Mm.202728: expressed sequence AI447901
- Mm.202696: expressed sequence AA409221
- Mm.201729: expressed sequence AI426007
- Mm.2011: glutathione S-transferase, mu 1
- Mm.200858: RIKEN cDNA 2410129E14 gene
- Mm.200843: synuclein, beta
- Mm.200817: expressed sequence AW124717
- Mm.200817: expressed sequence AW124717
- Mm.200806: (Manual) no clear assignment, probably non-coding (but spliced) RNA gene
- Mm.200511: expressed sequence AI115024
- Mm.199903: expressed sequence AI850290
- Mm.199652: expressed sequence AI838505
- Mm.198588: expressed sequence AI851970
- Mm.19834: RIKEN cDNA 0610033L03 gene
- Mm.197523: brain acyl-CoA hydrolase
- Mm.196614: eukaryotic translation elongation factor 1 alpha 1
- Mm.196611: synapsin I
- Mm.196607: eukaryotic translation initiation factor 5A
- Mm.196605: hexokinase 1
- Mm.196578: mitochondrial carrier homolog 1
- Mm.196344: lusterin
- Mm.196239: RIKEN cDNA 4922501H04 gene
- Mm.195869: ATPase, H+ transporting, lysosomal 31kDa, V1 subunit E
- Mm.1956: neurofilament, light polypeptide
- Mm.19370: ATP synthase, H+ transporting, mitochondrial F1F0 complex, subunit e
- Mm.193539: H1 histone family, member 2
- Mm.192991: Mus musculus, Similar to metallothionein 1, clone MGC:27821 IMAGE:3483861, mRNA, complete cds
- Mm.19133: amyloid beta (A4) precursor-like protein 2
- Mm.19047: expressed sequence AI425998
- Mm.182912: growth hormone inducible transmembrane protein
- Mm.18218: ganglioside-induced differentiation-associated-protein 1
- Mm.181894: RIKEN cDNA 2900092E17 gene
- Mm.181721: RIKEN cDNA 2610041P16 gene
- Mm.180182: cytochrome c oxidase, subunit Vb
- Mm.1776: ferritin heavy chain
- Mm.177272: brain protein 17
- Mm.177117: Mus musculus, clone MGC:31632 IMAGE:4511454, mRNA, complete cds
- Mm.176927: RIKEN cDNA 2610301115 gene
- Mm.17484: synuclein, alpha
- Mm.16831: creatine kinase, brain
- Mm.16769: RIKEN cDNA 5031406P05 gene
- Mm.16767: heterogeneous nuclear ribonucleoprotein A2/B1
- Mm.16763: aldolase 1, A isoform
- Mm.16228: solute carrier family 25 (mitochondrial carrier; adenine nucleotide translocator), member 4
- Mm.16080: dynamin
- Mm.158871: RIKEN cDNA 2410003L22 gene
- Mm.157929: ESTs, Weakly similar to PBAS MOUSE PROBASIN PRECURSOR
- Mm.157859: ESTs
- Mm.157648: RIKEN cDNA 5730403B10 gene
- Mm.15711: cyclic nucleotide phosphodiesterase 1
- Mm.156959: beta-spectrin 4
- Mm.15571: amyloid beta (A4) precursor protein
- Mm.15512: potassium voltage-gated channel, shaker-related subfamily, beta member 2
- Mm.154651: purine rich element binding protein B
- Mm.153758: RIKEN cDNA 0610040H15 gene
- Mm.15125: stromal cell derived factor receptor 1
- Mm.14798: ribosomal protein S13
- Mm.142511: expressed sequence AI173355
- Mm.142187: RIKEN cDNA 2610009E16 gene
- Mm.142140: neurofilament, medium polypeptide
- Mm.140761: DnaJ (Hsp40) homolog, subfamily C, member 5
- Mm.139797: expressed sequence AI848587
- Mm.139239: RIKEN cDNA 2900016C05 gene
- Mm.139239: RIKEN cDNA 2900016C05 gene
- Mm.139239: RIKEN cDNA 2900016C05 gene
- Mm.138866: apolipoprotein E
- Mm.13859: ribosomal protein L41
- Mm.1383: Rho GDP dissociation inhibitor (GDI) gamma
- Mm.135621: expressed sequence AI848120
- Mm.13445: 3-oxoacid CoA transferase
- Mm.1339: chromogranin B
- Mm.131127: RIKEN cDNA 6230410L23 gene
- Mm.12958: kinesin light chain 2
- Mm.12860: G protein-coupled receptor 37-like 1
- Mm.1268: proteolipid protein (myelin)
- Mm.1268: (Manual assignment) PLP Myelin Proteolipid Protein, uh05d10.r1 Soares mouse hypothalamus NMHy Mus musculus cDNA clone 1617043 5' similar to gb:M54927 MYELIN PROTEOLIPID PROTEIN
- Mm.12468: glioblastoma amplified sequence
- Mm.124592: expressed sequence AW214631
- Mm.1239: glial fibrillary acidic protein
- Mm.1222: brain abundant, membrane attached signal protein 2
- Mm.115124: brain protein 14
- Mm.114810: expressed sequence AW060990
- Mm.1147: Mus musculus calmodulin III (Calm3) mRNA, 3' untranslated region
- Mm.10727: ATPase, H+ transporting, lysosomal 56/58kD, V1 subunit B, isoform 2
- Mm.103709: potassium inwardly-rectifying channel, subfamily J, member 10
- Mm.103605: DnaJ (Hsp40) homolog, subfamily B, member 10
- Mm.102278: secretory carrier membrane protein 5
- Mm.102244: expressed sequence R74975
- Mm.101476: (Manual assignment) BNPI, VGLUT-1, mouse homolog of putative vesicular glutamate transporter, Na+/Phosphate cotransporter
- Mm.100980: calneuron 1
- Mm.1008: prostaglandin D2 synthase (21 kDa, brain)
- Mm.1008: (Manual) Prostaglandin H2 D-Isomerase (PGD2 SYNTHASE) (PGDS2) (PGDS) member of lipocalin family

### Liste D: Positivmarker neurale Stammzellen (1.);

### ES-Zellen +; PSA-NCAM - ; Adult brain -

- Mm.9703: (Manual) copper transport protein/chaperone ATOX1
- Mm.930: cathepsin L
- Mm.90787: nerve growth factor receptor (TNFRSF16) associated protein 1
- Mm.90587: enolase 1, alpha non-neuron
- Mm.90115: lysophospholipase 1
- Mm.90003: gap junction membrane channel protein beta 3
- Mm.88302: EST, Weakly similar to S14234 hypothetical protein - mouse
- Mm.88212: tubulin, alpha 6
- Mm.87581: (Manual) fibronectin 1, internal tag (major tag probably AAAAAAAAAAA)
- Mm.87293: WD repeat domain 12
- Mm.87216: Rab geranylgeranyl transferase, a subunit
- Mm.8155: TG interacting factor
- Mm.78861: nucleolar and coiled-body phosphoprotein 1
- Mm.76780: ESTs
- Mm.7417: cyclin D3
- Mm.7387: RNA polymerase 1-4 (194 kDa subunit)
- Mm.7381: hypoxia induced gene 1
- Mm.725: ribosomal protein L7a
- Mm.71046: ESTs
- Mm.70127: ribosomal protein L12
- Mm.69647: pancreas specific transcription factor, 1a
- Mm.69049: cDNA sequence AF155546
- Mm.6700: eukaryotic translation initiation factor 4E binding protein 1
- Mm.66: ribosomal protein S4, X-linked
- Mm.6579: centromere autoantigen A
- Mm.6534: calpain, small subunit 1
- Mm.6343: nucleophosmin 1
- Mm.584: annexin A2
- Mm.57223: helicase, lymphoid specific
- Mm.57153: sterol O-acyltransferase 2
- Mm.5624: DEAD/H (Asp-Glu-Ala-Asp/His) box polypeptide 16
- Mm.548: cytochrome c oxidase, subunit VIc
- Mm.5305: (Manual) GNB2L1, RACK1, Receptor of activated C kinase, WD40-repeat protein
- Mm.5290: (Manual) 60S ribosomal protein L17 (L23) (popey3-annotation wrong)
- Mm.4993: matrix metalloproteinase 3
- Mm.493: CCCTC-binding factor
- Mm.4890: Finkel-Biskis-Reilly murine sarcoma virus (FBR-MuSV) ubiquitously expressed (fox derived)
- Mm.4770: frizzled homolog 7 (Drosophila)
- Mm.4742: proliferation-associated 2G4, 38kD
- Mm.46461: L-threonine dehydrogenase
- Mm.4606: branched chain aminotransferase 1, cytosolic
- Mm.4560: low density lipoprotein receptor-related protein associated protein 1
- Mm.45237: RIKEN cDNA 2610318N02 gene
- Mm.45151: RIKEN cDNA 1700043E15 gene
- Mm.4502: mini chromosome maintenance deficient (S. cerevisiae)
- Mm.43831: lectin, galactose binding, soluble 1
- Mm.43162: RIKEN cDNA 0710008D09 gene
- Mm.42960: RIKEN cDNA 2610301D06 gene
- Mm.4280: RIKEN cDNA 2010203J19 gene
- Mm.42790: ribosomal protein S18
- Mm.42767: ribosomal protein S17
- Mm.42197: proteasome (prosome, macropain) subunit, beta type 1
- Mm.42196: nuclear protein 95
- Mm.42195: RuvB-like protein 1
- Mm.41467: Mus musculus, clone MGC:28892 IMAGE:4912251, mRNA, complete cds
- Mm.41151: ESTs
- Mm.41061: RIKEN cDNA 1810055P05 gene
- Mm.41: (Manual) Mitochondrial ATP synthase oligomycin sensitivity conferral protein (OSCP) (ATP5O)
- Mm.4095: inactive X specific transcripts
- Mm.4024: cofilin 1, non-muscle
- Mm.3925: S100 calcium binding protein A4
- Mm.38718: ESTs, Moderately similar to S12207 hypothetical protein
- Mm.3845: Mus musculus, eukaryotic translation termination factor 1, clone MGC: 18745 IMAGE:3992883, mRNA, complete cds
- Mm.3845: Mus musculus, eukaryotic translation termination factor 1, clone MGC:18745 IMAGE:3992883, mRNA, complete cds
- Mm.38151: adenylosuccinate lyase
- Mm.38057: ESTs
- Mm.3776: Mus musculus, clone MGC:37810 IMAGE:5098241, mRNA, complete cds
- Mm.3752: RAN binding protein 1
- Mm.36241: B-cell receptor-associated protein 37
- Mm.360: cytochrome c oxidase, subunit Va
- Mm.3572: RIKEN cDNA 1110033J19 gene
- Mm.35621: ESTs
- Mm.35605: cadherin 1
- Mm.3487: ribosomal protein L30
- Mm.3486: ribosomal protein L3
- Mm.34828: heat shock protein, 105 kDa
- Mm.34797: cellular retinoic acid binding protein I
- Mm.34606: RIKEN cDNA 2610511F02 gene
- Mm.34554: Mus musculus, Similar to E2F transcription factor 4, p107/p130-binding, clone MGC:37558 IMAGE:4987691, mRNA, complete cds
- Mm.3438: lamin A
- Mm.34351: Mus musculus, Similar to hypothetical protein FLJ13187, clone MGC:28979 IMAGE:4503757, mRNA, complete cds
- Mm.34102: ornithine decarboxylase, structural
- Mm.3379: serine hydroxymethyl transferase 1 (soluble)
- Mm.33240: epithelial V-like antigen
- Mm.33202: RIKEN cDNA 2410018A17 gene
- Mm.32879: testis expressed gene 17
- Mm.321: secreted phosphoprotein 1
- Mm.318: RIKEN cDNA 2010107E04 gene
- Mm.31227: expressed sequence AW123847
- Mm.30929: peroxiredoxin 1
- Mm.3049: CDC28 protein kinase 1
- Mm.30242: peptidylprolyl isomerase D (cyclophilin D)
- Mm.30184: RIKEN cDNA 2700086I23 gene
- Mm.30114: amyotrophic lateral sclerosis 2 (juvenile) homolog (human)
- Mm.30060: RIKEN cDNA 2310008N12 gene
- Mm.30049: complement component 1, q subcomponent binding protein
- Mm.30034: translocase of inner mitochondrial membrane 8 homolog a (yeast)
- Mm.29904: mitochondrial ribosomal protein L15
- Mm.29902: Mus musculus, Similar to phosphoserine aminotransferase, clone MGC:6462 IMAGE:2616298, mRNA, complete cds
- Mm.29859: eukaryotic translation initiation factor 2, subunit 2 (beta, 38kDa)
- Mm.29856: RIKEN cDNA 9130022B02 gene
- Mm.29717: 3-monooxgenase/tryptophan 5-monooxgenase activation protein, gamma polypeptide
- Mm.29714: (Manual) mouse version of muscle-specific protein M9
- Mm.29675: thioredoxin-like 2
- Mm.29619: RIKEN cDNA 1200007E24 gene
- Mm.29513: NADH dehydrogenase (ubiquinone) 1 alpha subcomplex, 7 (14.5kD, B14.5a)
- Mm.29504: sperm specific antigen 1
- Mm.2942: asparagine synthetase
- Mm.29405: ring-box 1
- Mm.29363: RIKEN cDNA 2310044F10 gene
- Mm.2930: Mus musculus, Similar to peter pan (Drosophila) homolog, clone MGC:25669 IMAGE:4489442, mRNA, complete cds
- Mm.29192: asparaginyl-tRNA synthetase
- Mm.29148: RIKEN cDNA 2400008B06 gene
- Mm.29122: RIKEN cDNA 0610012D09 gene
- Mm.29076: RIKEN cDNA 2510010F10 gene
- Mm.28919: destrin
- Mm.28892: expressed sequence AA959742
- Mm.28805: SET translocation
- Mm.2849: heat shock protein, 74 kDa, A
- Mm.28483: Mus musculus, Similar to hypothetical protein FLJ22479, clone IMAGE:4487274, mRNA, partial cds
- Mm.28405: serum/glucocorticoid regulated kinase
- Mm.28173: ESTs, Moderately similar to JC5224 methionine--tRNA ligase
- Mm.28053: RIKEN cDNA 1110017C15 gene
- Mm.28035: ESTs, Weakly similar to TRHY_HUMAN TRICHOHYALI
- Mm.27901: RIKEN cDNA 1110020J08 gene
- Mm.27858: RIKEN cDNA 1110036B12 gene
- Mm.27855: replication factor C (activator 1) 2 (40kD)
- Mm.2758: makorin, ring finger protein, 3
- Mm.27536: ESTs, Highly similar to hypothetical protein FLJ14075
- Mm.27526: (Manual) Arginyl tRNA synthetase (RIKEN cDNA 2610011N19)
- Mm.27186: Mus musculus, Similar to CG7083 gene product, clone MGC:6480 IMAGE:2646515, mRNA, complete cds
- Mm.2718: eukaryotic translation elongation factor 1 beta 2
- Mm.2718: eukaryotic translation elongation factor 1 beta 2
- Mm.27134: RIKEN cDNA 2610033C09 gene
- Mm.265: ribosomal protein S25
- Mm.2647: profilin 1
- Mm.2623: serine (or cysteine) proteinase inhibitor, clade B (ovalbumin), member 6
- Mm.25642: RIKEN cDNA 2310034K10 gene
- Mm.254: tumor protein, translationally-controlled 1
- Mm.25328: ESTs
- Mm.24513: solute carrier family 25 (mitochondrial carrier; adenine nucleotide translocator), member 13
- Mm.24506: Mus musculus, clone IMAGE:3591061, mRNA, partial cds
- Mm.2437: BING4 protein
- Mm.2424: ribosomal protein L10A
- Mm.24220: RIKEN cDNA 2310003F16 gene
- Mm.24219: RIKEN cDNA 1810037117 gene
- Mm.24174: Mus musculus, similar to alanyl-tRNA synthetase (H. sapiens), clone MGC:37368 IMAGE:4976684, mRNA, complete cds
- Mm.2395: male enhanced antigen 1
- Mm.2355: prohibitin
- Mm.235: ubiquitin B
- Mm.22731: integrin beta 4 binding protein
- Mm.22626: Mus musculus, Similar to chromosome 14 open reading frame 3, clone MGC:36589 IMAGE:5320590, mRNA, complete cds
- Mm.2246: proteasome (prosome, macropain) subunit, beta type 7
- Mm.22421: telomerase binding protein, p23
- Mm.22421: telomerase binding protein, p23
- Mm.22317: RIKEN cDNA 8430410A17 gene
- Mm.22288: cyclin D1
- Mm.22271: smt3-specific isopeptidase 1
- Mm.220992: Mus musculus, clone IMAGE:3492506, mRNA, partial cds
- Mm.219671: Mus musculus, clone MGC:36369 IMAGE:4982239, mRNA, complete cds
- Mm.219458: RNA binding protein gene with multiple splicing
- Mm.218533: RIKEN cDNA 1500016H10 gene
- Mm.2180: heat shock protein, 84 kDa 1
- Mm.21758: cytochrome P450, 2e1, ethanol inducible
- Mm.21630: expressed sequence AU022237
- Mm.21569: RIKEN cDNA 2700069E09 gene
- Mm.213020: (Manual) 60S ribosomal protein L32 (RPL32)
- Mm.212899: Mus musculus, Similar to RIKEN cDNA 1200009K13 gene, clone MGC: 18794 IMAGE:4193513, mRNA, complete cds
- Mm.21289: ribosomal protein S12
- Mm.21086: eukaryotic translation elongation factor 1 delta (guanine nucleotide exchange protein)
- Mm.210638: EST
- Mm.21062: expressed sequence C87860
- Mm.21054: nuclease sensitive element binding protein 1
- Mm.20943: FK506 binding protein 9
- Mm.20925: G1 to phase transition 1
- Mm.20918: nuclear localization signal protein absent in velo-cardio-facial patients
- Mm.20848: regulatory factor X-associated ankyrin-containing protein
- Mm.20847: sorting nexin 5
- Mm.20294: selenophosphate synthetase 2
- Mm.20290: expressed sequence AW536573
- Mm.20288: glutathione reductase 1
- Mm.200920: ribosomal protein S28
- Mm.197601: heat shock 10 kDa protein 1 (chaperonin 10)
- Mm.197555: hypothetical protein MGC6664
- Mm. 197551: heat shock 70kD protein 8
- Mm.196604: angio-associated migratory protein, related sequence
- Mm.196586: cullin 2
- Mm. 196581: mitogen activated protein kinase 1
- Mm.196526: ADP-ribosylation factor 6
- Mm.196396: tubulin, alpha 1
- Mm.196081: peptidylprolyl isomerase (cyclophilin)-like 1
- Mm.196: neural precursor cell expressed, developmentally down-regulated gene 8
- Mm.195894: Mus musculus, clone MGC:11792 IMAGE:3595167, mRNA, complete cds
- Mm.19169: thioredoxin-like (32kD)
- Mm.188: (Manual) X-linked phosphoglycerate kinase (PGK1)
- Mm.18637: teratocarcinoma expressed, serine rich
- Mm. 18459: fibroblast growth factor inducible 14
- Mm.183022: DNA segment, Chr 8, Brigham & Women's Genetics 1112 expressed
- Mm.182951: proteasome (prosome, macropain) subunit, alpha type 2
- Mm.182931: phosphoribosylaminoimidazole carboxylase, phosphoribosylaminoribosylaminoimidazole, succinocarboxamide synthetase
- Mm.182471: RIKEN cDNA 2610524G07 gene
- Mm.181765: Mus musculus 8 days embryo whole body cDNA, RIKEN full-length enriched library, clone:5730409M10:CCAAT/enhancer binding protein alpha (C/EBP), related sequence 1, full insert sequence
- Mm.181740: interferon-related developmental regulator 2
- Mm.180299: DNA segment, Chr 16, Wayne State University 109, expressed
- Mm.17932: purine-nucleoside phosphorylase
- Mm.1777: heat shock protein, 60 kDa
- Mm.176845: RIKEN cDNA 1110069M14 gene
- Mm.175848: (Manual) small Ca-binding protein Calgizzarin (S100A11) (ENDOTHELIAL MONOCYTE-ACTIVATING POLYPEPTIDE) (EMAP)
- Mm.175661: RIKEN cDNA 1110036C17 gene
- Mm.1710: hydroxymethylbilane synthase
- Mm.17031: POU domain, class 5, transcription factor 1
- Mm.16757: solute carrier family 20, member 1
- Mm.1639: myeloid cell leukemia sequence 1
- Mm.16110: cyclin E1
- Mm.157778: RIKEN cDNA 2610034E13 gene
- Mm.154915: ribosomal protein S29
- Mm.154387: transketolase
- Mm.153963: CD8 antigen, beta chain
- Mm.153159: chaperonin subunit 6a (zeta)
- Mm.152291: EST
- Mm.151329: karyopherin (importin) beta 3
- Mm.148973: RIKEN cDNA 3010025E17 gene
- Mm.147946: MYB binding protein (P160) 1a
- Mm.147693: ribosomal protein S3
- Mm.14768: reduced expression 3
- Mm.14663: ATP synthase, H+ transporting, mitochondrial F0 complex, subunit g
- Mm.143141: eukaryotic translation initiation factor 1A
- Mm.142740: metallothionein 2
- Mm.14245: ribosomal protein, large P2
- Mm.14244: ribosomal protein L9
- Mm.141443: lactate dehydrogenase 1, A chain
- Mm.141187: trans-golgi network protein 2
- Mm.140380: ribosomal protein L23
- Mm.139825: Mus musculus, Similar to xylosylprotein betal,4-galactosyltransferase, polypeptide 7 (galactosyltransferase I), clone MGC: 28643 IMAGE:4224150, mRNA, complete cds
- Mm.13705: (Manual) mouse version of p180 ribosome receptor/ribosome binding protein 1 RRBP1
- Mm.13020: ribosomal protein L13a
- Mm.12909: amyloid beta (A4) precursor protein-binding, family A, member 3
- Mm.1275: thioredoxin 1
- Mm.12508: karyopherin (importin) alpha 2
- Mm.1164: SEC61, gamma subunit (S. cerevisiae)
- Mm.11376: ribosomal protein L36
- Mm.1125: expressed in non-metastatic cells 2, protein (NM23B) (nucleoside diphosphate kinase)
- Mm.1120: endometrial bleeding associated factor
- Mm.108076: phosphofructokinase, platelet
- Mm.10706: RIKEN cDNA 2010004J23 gene
- Mm.10706: (Manual) mouse version of 60S ribosomal protein L4
- Mm.10702: calcyclin binding protein
- Mm.10665: Mus musculus, clone IMAGE:3498496, mRNA, partial cds
- Mm.10623: expressed sequence AI480570
- Mm.10600: glutamate dehydrogenase
- Mm.1056: solute carrier family 1, member 7
- Mm.10474: RIKEN cDNA 3110005M08 gene
- Mm.101619: EST
- Mm.10: spermidine synthase
- Mm.4325: Kruppel-like factor 4 (gut) [Swissprot: sp|Q60793;sp|Q9R255;]
- Mm.12919: insulin-like growth factor 2, binding protein 1 [Swissprot: sp|O88477;]
- Mm.20348: nidogen 2 [Swissprot: sp|O88322;sp|Q8R5G0;sp|Q9CT94;]
- Mm.34407: MAD homolog 7 (Drosophila) [Swissprot: sp|O35253;sp|Q9CSC7;]
- Mm.4451: hairy and enhancer of split 1, (Drosophila) [Swissprot: none]
- Mm.57195: nodal [Swissprot: sp|P43021;]
- Mm.1249: laminin, gamma 1 [Swissprot: sp|P02468;]
- Mm.27706: ash2 (absent, small, or homeotic)-like (Drosophila) [Swissprot:
- Mm.4603: scavenger receptor class B1 [Swissprot: sp|Q61009;sp|Q9CWJ7;]
- Mm.181562: adhesion regulating molecule 1 [Swissprot: sp|Q8VCI8;sp|Q922A7;sp|Q9JKV1;]
- Mm.43444: MAD2 (mitotic arrest deficient, homolog)-like 1 (yeast) [Swissprot:
- Mm.103675: sacsin [Swissprot: none]
- Mm.980: tenascin C [Swissprot: sp|Q64706;sp|Q9WUU4;]
- Mm.5090: cripto, teratocarcinoma-derived growth factor (Tdgf1)
- Mm.30177: D11Ertd603e, DNA segment, Chr 11, ERATO Doi 603
- Mm.233844: C330012H03Rik, RIKEN cDNA C330012H03

### Liste E: Positivmarker neurale Stammzellen (2.);

### ES-Zellen +; PSA-NCAM -/+; Adult brain -

- Mm.99776: cytosolic aminopeptidase P
- Mm.9916: RIKEN cDNA 1200008012 gene
- Mm.99: ribonucleotide reductase M2
- Mm.9811: RIKEN cDNA 2310008M10 gene
- Mm.9257: (Manual) uncharacterized protein corresponding to human sp|Q9Y3I0, similar to E.coli rtcB, UPF0027-family
- Mm.925: transcription factor Dp 1
- Mm.918: heat shock 70kD protein 5 (glucose-regulated protein, 78kD)
- Mm.911: high mobility group nucleosomal binding domain 2
- Mm.9043: heterogeneous nuclear ribonucleoprotein L
- Mm.89927: signal recognition particle 9 kDa
- Mm.89579: mannose-P-dolichol utilization defect 1
- Mm.89136: H3 histone, family 3A
- Mm.88212: tubulin, alpha 6
- Mm.880: mammary tumor integration site 6
- Mm.8552: baculoviral IAP repeat-containing 5
- Mm.8256: KH domain containing, RNA binding, signal transduction associated 1
- Mm.8155: TG interacting factor
- Mm.78861: nucleolar and coiled-body phosphoprotein 1
- Mm.7793: protein phosphatase 1, catalytic subunit, gamma isoform
- Mm.7723: poly(A) binding protein, nuclear 1
- Mm.76278: RIKEN cDNA 2610203K23 gene
- Mm.7516: nuclear autoantigenic sperm protein (histone-binding)
- Mm.7312: DNA segment, Chr 17, human D6S56E 2
- Mm.7141: proliferating cell nuclear antigen
- Mm.6787: splicing factor, arginine/serine-rich 3 (SRp20)
- Mm.66: ribosomal protein S4, X-linked
- Mm.6476: RIKEN cDNA 2700084L22 gene
- Mm.64104: RIKEN cDNA 2410016F19 gene
- Mm.6343: nucleophosmin 1
- Mm.61901: expressed sequence AI429604
- Mm.6065: inosine 5'-phosphate dehydrogenase 2
- Mm.5624: DEAD/H (Asp-Glu-Ala-Asp/His) box polypeptide 16
- Mm.548: cytochrome c oxidase, subunit VIc
- Mm.5305: guanine nucleotide binding protein, beta 2, related sequence 1
- Mm.525: eukaryotic translation initiation factor 4, gamma 2
- Mm.5114: dishevelled 2, dsh homolog (Drosophila)
- Mm.4933: mini chromosome maintenance deficient 6 (S. cerevisiae)
- Mm.4890: Finkel-Biskis-Reilly murine sarcoma virus (FBR-MuSV) ubiquitously expressed (fox derived)
- Mm.4846: lamin B1
- Mm.4756: leptin receptor
- Mm.46754: expressed sequence AI316867
- Mm.46533: RIKEN cDNA 1110007L15 gene
- Mm.4551: villin 2
- Mm.4550: ATPase, Na+/K+ transporting, beta 1 polypeptide
- Mm.4541: SRY-box containing gene 2
- Mm.45312: anaphase-promoting complex subunit 5
- Mm.45149: ESTs
- Mm.45132: expressed sequence AW121759
- Mm.4426: Cd63 antigen
- Mm.43444: MAD2 (mitotic arrest deficient, homolog)-like 1 (yeast)
- Mm.4280: RIKEN cDNA 2010203J19 gene
- Mm.42767: ribosomal protein S17
- Mm.4237: topoisomerase (DNA) II alpha
- Mm.42197: proteasome (prosome, macropain) subunit, beta type 1
- Mm.4215: catalase 1
- Mm.41940: RIKEN cDNA 6530409L22 gene
- Mm.4189: cyclin A2
- Mm.41023: RIKEN cDNA 1110021E09 gene
- Mm.4078: antigen identified by monoclonal antibody Ki 67
- Mm.4071: laminin receptor 1 (67kD, ribosomal protein SA)
- Mm.4024: cofilin 1, non-muscle
- Mm.3931: Max protein
- Mm.38930: expressed sequence AA407558
- Mm.38912: RIKEN cDNA 2410129H14 gene
- Mm.38611: RIKEN cDNA 2210021A15 gene
- Mm.38528: RIKEN cDNA 2810430M08 gene
- Mm.38306: macrophage erythroblast attacher
- Mm.3797: nucleosome assembly protein 1-like 1
- Mm.37835: ribosomal protein L7
- Mm.372: ribosomal protein S26
- Mm.36511: mitochondrial ribosomal protein L32
- Mm.35844: growth arrest specific 5
- Mm.35829: erythroid differentiation regulator
- Mm.35661: Mus musculus, Similar to hypothetical protein, clone MGC:29235 IMAGE: 5043282, mRNA, complete cds
- Mm.35087: expressed sequence AA673488
- Mm.3501: kinesin family member C5A
- Mm.34914: ESTs
- Mm.3487: ribosomal protein L30
- Mm.3444: bromodomain-containing 2
- Mm.34385: expressed sequence AI450344
- Mm.34261: expressed sequence AW557761
- Mm.3381: ribosomal protein S8
- Mm.3380: kinesin family member 5B
- Mm.3360: tyrosine 3-monooxygenase/tryptophan 5-monooxygenase activation protein, zeta polypeptide
- Mm.326: RIKEN cDNA 1110038L14 gene
- Mm.320: DNA polymerase alpha 2, 68 kDa
- Mm.3199: RIKEN cDNA 1500001N04 gene
- Mm.31512: ring finger protein 2
- Mm.31228: RIKEN cDNA 1810022K09 gene
- Mm.30806: ribosomal protein L19
- Mm.3054: alpha-L-iduronidase
- Mm.3035: RIKEN cDNA 3110006P09 gene
- Mm.30270: proteasome (prosome, macropain) subunit, alpha type 4
- Mm.30120: ribosomal protein S27-like
- Mm.30069: RIKEN cDNA 1200003J11 gene
- Mm.30011: ribosomal protein S23
- Mm.29931: cell division cycle 20 homolog (S. cerevisiae)
- Mm.29923: SMT3 (supressor of mif two, 3) homolog 2 (S. cerevisiae)
- Mm.29911: RIKEN cDNA 3200001M24 gene
- Mm.29896: ribosomal protein L21
- Mm.2986: expressed sequence AW146116
- Mm.29829: expressed sequence AI326010
- Mm.29666: solute carrier family 25 (mitochondrial carnitine/acylcarnitine translocase), member 20 ,
- Mm.2966: isocitrate dehydrogenase 2 (NADP+), mitochondrial
- Mm.29296: RIKEN cDNA 1110003H02 gene
- Mm.29194: RIKEN cDNA 1700094M07 gene
- Mm.29133: budding uninhibited by benzimidazoles 1 homolog, beta (S. cerevisiae)
- Mm.29122: RIKEN cDNA 0610012D09 gene
- Mm.29055: chromobox homolog 1 (Drosophila HP1 beta)
- Mm.29054: RIKEN cDNA 2610529I12 gene
- Mm.29005: expressed sequence AU021749
- Mm.28995: RIKEN cDNA 2010009J12 gene
- Mm.28985: ribosomal protein L27
- Mm.28965: RIKEN cDNA 0710007A14 gene
- Mm.28964: Mus musculus, clone IMAGE:4949762, mRNA, partial cds
- Mm.28961: cleavage and polyadenylation specific factor 5, 25 kD subunit
- Mm.28909: protein tyrosine phosphatase 4a1
- Mm.28899: RIKEN cDNA 1110059P08 gene
- Mm.28805: SET translocation
- Mm.28805: SET translocation
- Mm.28805: SET translocation
- Mm.28726: EST C77032
- Mm.28694: RIKEN cDNA 2410088K19 gene
- Mm.28560: Ly1 antibody reactive clone
- Mm.28499: Mus musculus, similar to CG15881 gene product (H. sapiens), clone MGC:36308 IMAGE:5040108, mRNA, complete cds
- Mm.28299: ESTs, Highly similar to GUAA_HUMAN GMP SYNTHASE
- Mm.28222: RIKEN cDNA 2610307C23 gene
- Mm.28121: RIKEN cDNA 1110061A19 gene
- Mm.28044: filamin-like protein
- Mm.27972: NS1-associated protein 1
- Mm.27927: heterogeneous nuclear ribonucleoprotein A1
- Mm.27852: expressed sequence AW555814
- Mm.27818: eukaryotic translation elongation factor 2
- Mm.27796: RIKEN cDNA 5730427N09 gene
- Mm.27669: small nuclear ribonucleoprotein E
- Mm.27660: RIKEN cDNA 5730420G12 gene
- Mm.27624: RIKEN cDNA C530002L11 gene
- Mm.27293: RIKEN cDNA 4833420K19 gene
- Mm.27269: RIKEN cDNA 2310037I24 gene
- Mm.27141: Rac GTPase-activating protein 1
- Mm.27074: RIKEN cDNA 2610019N13 gene
- Mm.265: ribosomal protein S25
- Mm.2591: RNA binding motif protein 3
- Mm.25558: RIKEN cDNA 2410018J24 gene
- Mm.25542: (Manual) strange EST contig in intron of p137 (GPI-anchored transcytosis protein), maybe alternative C-terminus of sp|Q60865
- Mm.254: tumor protein, translationally-controlled 1
- Mm.25299: ESTs, Weakly similar to simple repeat sequence-containing transcript
- Mm.25164: gene trap locus 1-13
- Mm.25137: RIKEN cDNA 2410004B18 gene
- Mm.24870: (Manual assignment) UBP7 ubiquitin hydrolase
- Mm.24591: expressed sequence AW546279
- Mm.2424: ribosomal protein L10A
- Mm.24219: RIKEN cDNA 1810037I17 gene
- Mm.24042: RIKEN cDNA 1210001E11 gene
- Mm.23943: vesicle-associated membrane protein, associated protein A (33 kDa)
- Mm.23758: RIKEN cDNA 1110008P04 gene
- Mm.23695: dihydrofolate reductase
- Mm.23692: casein kinase II, alpha 1 related sequence 4
- Mm.23096: protein phosphatase 2 (formerly 2A), regulatory subunit B", alpha
- Mm.2287: proteasome (prosome, macropain) subunit, alpha type 5
- Mm.22731: integrin beta 4 binding protein
- Mm.2265: U1 small nuclear ribonucleoprotein 1C
- Mm.22387: expressed sequence AI314668
- Mm.22269: exportin 1, CRM1 homolog (yeast)
- Mm.22214: RIKEN cDNA 2610008F03 gene
- Mm.220918: heterogeneous nuclear ribonucleoprotein D-like
- Mm.220342: Mus musculus, clone IMAGE:3669867, mRNA, partial cds
- Mm.219670: Mus musculus, Similar to eukaryotic translation initiation factor 4 gamma, 1, clone IMAGE:4950789, mRNA, partial cds
- Mm.219668: RIKEN cDNA 2610209F03 gene
- Mm.219648: Mus musculus, Similar to nuclear matrix protein p84, clone MGC:28284 IMAGE:4010605, mRNA, complete cds
- Mm.21964: Mus musculus, clone IMAGE:3485208, mRNA, partial cds
- Mm.21873: retroviral integration site 1
- Mm.218657: cerebellar ataxia 3
- Mm.21841: splicing factor, arginine/serine-rich 2 (SC-35)
- Mm.218240: Mus musculus, clone IMAGE:5342828, mRNA, partial cds
- Mm.2180: heat shock protein, 84 kDa 1
- Mm.21764: small nuclear ribonucleoprotein polypeptide G
- Mm.21714: RIKEN cDNA 2410003A14 gene
- Mm.21559: non-POU-domain-containing, octamer binding protein
- Mm.213452: Mus musculus, clone IMAGE:5320271, mRNA, partial cds
- Mm.213020: (Manual) 60S ribosomal protein L32 (RPL32)
- Mm.21295: expressed sequence AW214031
- Mm.21289: ribosomal protein S12
- Mm.21281: ring finger protein 4
- Mm.21185: adaptor-related protein complex AP-3, beta 1 subunit
- Mm.2115: heterogeneous nuclear ribonucleoprotein U
- Mm.21094: DNA segment, Chr 9, Wayne State University 138, expressed
- Mm.21054: nuclease sensitive element binding protein 1
- Mm.20927: transforming growth factor beta 1 induced transcript 4
- Mm.206399: ESTs
- Mm.2038: Ras-GTPase-activating protein SH3-domain binding protein
- Mm.2025: survival motor neuron
- Mm.200837: Mus musculus, clone IMAGE:5355658, mRNA
- Mm.196614: eukaryotic translation elongation factor 1 alpha 1
- Mm.196608: expressed sequence AA407306
- Mm.196526: ADP-ribosylation factor 6
- Mm.196515: DNA segment, Chr 1, ERATO Doi 692, expressed
- Mm.196396: tubulin, alpha 1
- Mm.196365: RIKEN cDNA 4833416109 gene
- Mm.196328: RIKEN cDNA 5830466J11 gene
- Mm.195898: phosphatidylethanolamine binding protein
- Mm.1951: ribonucleic acid binding protein S1
- Mm.1948: t-complex testis expressed 1
- Mm.193688: RIKEN cDNA 2700059D21 gene
- Mm.19187: prothymosin alpha
- Mm.19101: DEAD (aspartate-glutamate-alanine-aspartate) box polypeptide 5
- Mm.19015: serine racemase
- Mm.18923: mini chromosome maintenance deficient 7 (S. cerevisiae)
- Mm.18921: valosin containing protein
- Mm. 18856: mitogen-activated protein kinase 6
- Mm.18705: vacuolar protein sorting 4b (yeast)
- Mm.18700: RIKEN cDNA 1200009K13 gene
- Mm.18637: teratocarcinoma expressed, serine rich
- Mm.18516: H3 histone, family 3B
- Mm.1843: heat shock protein, 86 kDa 1
- Mm.183102: actin-related protein 3 homolog (yeast)
- Mm.183016: thymine DNA glycosylase
- Mm.181880: RIKEN cDNA 1110007A14 gene
- Mm.181562: adhesion regulating molecule 1
- Mm.1815: cytidine 5'-triphosphate synthase
- Mm.180873: RIKEN cDNA 2510019J09 gene
- Mm.180873: (Manual) probably reverse tag of 60S ribosomal protein L18a
- Mm.180409: ubiquitin-conjugating enzyme E2H
- Mm.180271: RIKEN cDNA 5630400D24 gene
- Mm.17989: chaperonin subunit 8 (theta)
- Mm.1777: heat shock protein, 60 kDa
- Mm.1775: hematological and neurological expressed sequence 1
- Mm.177451: RIKEN cDNA 5730544L10 gene
- Mm.17330: ESTs
- Mm.17306: tropomyosin 3, gamma
- Mm.1703: tubulin, beta 5
- Mm.16976: TAF9 RNA polymerase II, TATA box binding protein (TBP)-associated factor, 32 kDa
- Mm.16775: ribosomal protein S24
- Mm.16767: heterogeneous nuclear ribonucleoprotein A2/B1
- Mm.16711: mini chromosome maintenance deficient 2 (S. cerevisiae)
- Mm.16525: polo-like kinase homolog, (Drosophila)
- Mm.1639: myeloid cell leukemia sequence 1
- Mm.16323: eukaryotic translation initiation factor 4A2
- Mm.16323: eukaryotic translation initiation factor 4A2
- Mm.156892: heterogeneous nuclear ribonucleoprotein D
- Mm.15571: amyloid beta (A4) precursor protein
- Mm.154915: ribosomal protein S29
- Mm.153457: RIKEN cDNA 2810406C15 gene
- Mm.148973: RIKEN cDNA 3010025E17 gene
- Mm.142872: heterogeneous nuclear ribonucleoprotein K
- Mm.14245: ribosomal protein, large P2
- Mm.14244: ribosomal protein L9
- Mm.142363: RIKEN cDNA 2810036L13 gene
- Mm.140804: Mus musculus, guanine nucleotide binding protein (G protein), gamma 5, clone MGC:8292 IMAGE:3593324, mRNA, complete cds
- Mm.140380: ribosomal protein L23
- Mm.13886: suppressor of initiator codon mutations, related sequence 1 (S. cerevisiae)
- Mm.133825: RIKEN cDNA 0610010123 gene
- Mm.13356: RIKEN cDNA 3110079L04 gene
- Mm.131705: Mus musculus, Similar to single-stranded DNA binding protein, clone MGC:41439 IMAGE: 1314987, mRNA, complete cds
- Mm.12858: eukaryotic translation initiation factor 4A1
- Mm.12706: Mus musculus, Similar to CG11246 gene product, clone MGC:8248 IMAGE:3591968, mRNA, complete cds
- Mm.12604: sirtuin 1 ((silent mating type information regulation 2, homolog) 1 (S. cerevisiae)
- Mm.12568: expressed sequence AW541137
- Mm.12508: karyopherin (importin) alpha 2
- Mm.12441: expressed sequence AU014645
- Mm.124: thymopoietin
- Mm.12236: zinc finger protein 207
- Mm.12145: retinoblastoma binding protein 4
- Mm.116989: actin-like
- Mm.111: poly(rC) binding protein 2
- Mm.10706: RIKEN cDNA 2010004J23 gene
- Mm.10474: RIKEN cDNA 3110005M08 gene
- Mm.10409: golgi autoantigen, golgin subfamily a, 4
- Mm.103675: sacsin
- Mm.1013: ligase I, DNA, ATP-dependent
- Mm.101274: RIKEN cDNA 2010008E23 gene
- Mm.10076: mitochondrial ribosomal protein L13
- Mm.16469: Nmycl, neuroblastoma myc-related oncogene 1

## Patentansprüche

1. Zellpopulation, **dadurch gekennzeichnet, dass** mindestens 5% der Zellen neurale Vorläuferzellen sind, die wenigstens einen der in **Liste A** oder **Liste B** aufgeführten Marker aufweisen.

2. Zellpopulation, **dadurch gekennzeichnet, dass** mindestens 5% der Zellen, neurale Vorläuferzellen sind, die wenigstens zwei, bevorzugt wenigstens 3 der in **Liste A** oder **Liste B** aufgeführten Marker aufweisen.

3. Zellpopulation, nach mindestens einem der Ansprüche 1 bis 2, **dadurch gekennzeichnet, dass** die neuralen Vorläuferzellen keinen in **Liste C** aufgeführten Marker aufweisen.

4. Zellpopulation nach mindestens einem der Ansprüche 1 bis 3, **dadurch gekennzeichnet, dass** mindestens 25 % der Zellen neurale Vorläuferzellen sind.

5. Zellpopulation nach mindestes einem der Ansprüche 1 bis 4, **dadurch gekennzeichnet, dass** es sich um eine murine Zellpopulation handelt und/oder die neuralen Vorläuferzellen aus Hirngewebe erhältlich ist.

6. Verfahren zur Isolierung einer Zellpopulation nach mindestens einem der Ansprüche 1 bis 5 mit folgenden Schritten:
a) Entnahme einer Probe aus dem Hirn
b) Isolieren der neuralen Vorläuferzellen unter Verwendung der angegebenen Marker
oder
a) Differenzierung von embryonalen Stammzellen zu neuralen Vorläuferzellen,
b) Isolieren der neuralen Vorläuferzellen unter Verwendung der angegebenen Marker
oder
a) Trans-Differenzierung von adulten, nicht neuralen Stammzellen zu neuralen Vorläuferzellen,
b) Isolieren der neuralen Vorläuferzellen unter Verwendung der angegebenen Marker
oder
a) Differenzierung von adulten, neuralen Stammzellen zu neuralen Vorläuferzellen,
b) Isolieren der neuralen Vorläuferzellen unter Verwendung der angegebenen Marker
oder
a) Differenzierung von immortalisierten Zellen zu neuralen Vorläuferzellen,
b) Isolieren der neuralen Vorläuferzellen unter Verwendung der angegebenen Marker.

7. Verwendung mindestens eines Markers ausgewählt aus der **Liste A** oder **Liste B** zu Identifizierung oder Isolierung von neuralen Vorläuferzellen.

8. Antikörper gegen einen Marker aus der **Liste A, B** oder **C**.

9. Diagnostikmittel enthaltend mindestens einen, bevorzugt zwei oder mehr Substanzen zur Erkennung der Marker der **Liste A, B** oder **C.**

10. Arzneimittel enthaltend die Zellpopulation nach einem der Ansprüche 1 bis 5.

11. Zellpopulation, **dadurch gekennzeichnet, dass** mindestens 5% der Zellen neurale Stammzellen sind, die wenigstens einen der in **Liste D** oder **Liste E** aufgeführten Marker aufweisen.

12. Zellpopulation, **dadurch gekennzeichnet, dass** mindestens 5% der Zellen neurale Stammzellen sind, die wenigstens zwei, bevorzugt wenigstens 3 der in **Liste D** oder **Liste E** aufgeführten Marker aufweisen.

13. Zellpopulation, nach mindestens einem der Ansprüche 11 bis 12, **dadurch gekennzeichnet, dass** die neuralen Stammzellen keinen in **Liste A** oder **Liste C** aufgeführten Marker aufweisen.

14. Zellpopulation nach mindestens einem der Ansprüche 11-13, **dadurch gekennzeichnet, dass** mindestens 25% der Zellen neurale Stammzellen sind.

15. Zellpopulation nach mindestes einem der Ansprüche 11 bis 14, **dadurch gekennzeichnet, dass** es sich um eine murine Zellpopulation handelt und/oder die neuralen Stammzellen aus Hirngewebe erhältlich.

16. Verfahren zur Isolierung einer Zellpopulation nach mindestens einem der Ansprüche 11 bis 15 mit folgenden Schritten:
a) Entnahme einer Probe aus dem Hirn
b) Isolieren der neuralen Stammzellen unter Verwendung der angegebenen Marker
oder
a) Differenzierung von embryonalen Stammzellen zu neuralen Stammzellen,
b) Isolieren der neuralen Stammzellen unter Verwendung der angegebenen Marker
oder
a) Trans-Differenzierung von adulten, nicht neuralen Stammzellen zu neuralen Stammzellen,
b) Isolieren der neuralen Stammzellen unter Verwendung der angegebenen Marker
oder
a) De-Differenzierung von adulten, neuralen Vorläuferzellen zu neuralen Stammzellen,
b) Isolieren der neuralen Stammzellen unter Verwendung der angegebenen Marker
oder
a) Differenzierung von immortalisierten Zellen zu neuralen Stammzellen,
b) Isolieren der neuralen Stammzellen unter Verwendung der angegebenen Marker.

17. Antikörper gegen einen Marker aus der **Liste D, E, A** oder **C**.

18. Diagnostikmittel enthaltend mindestens einen, bevorzugt zwei oder mehr Substanzen zur Erkennung der Marker der **Liste D, E, A** oder **C.**

19. Arzneimittel enthaltend die Zellpopulation nach einem der Ansprüche 11 bis 15.
